# EUROPEAN PATENT SPECIFICATION

(11) **EP 1 549 314 B1**
(45) Date of publication and mention of the grant of the patent: **12.12.2007**
(21) Application number: 03773199.9
(22) Date of filing: 07.10.2003
(51) Int. Cl.: A61K 31/498, A61P 25/28, A61P 25/16

(54) **USE OF BRIMONIDINE IN THE TREATMENT OF DEMENTIA AND PARKINSONS DISEASE**
VERWENDUNG VON BRIMONIDINE ZUR BEHANDLUNG VON DEMENTIA UND MORBUS PARKINSON
UTILISATION DE BRIMONIDINE DANS LE TRAITEMENT DE LA DEMENCE ET DE LA MALADIE DE PARKINSON

(30) Priority: 08.10.2002 US 417031 P
(43) Date of publication of application: 06.07.2005
(73) Proprietor: ALLERGAN, INC., Irvine, California 92612 (US)
(72) Inventor: TATTON, William, G., Fort MacLeod (CA); WHEELER, Larry, A., Irvine, CA 92612 (US); GIL, Daniel, W., Corona Del Mar, CA 92625 (US); DONELLO, John, E., Dana Point, CA 92629 (US)
(74) Representative: HOFFMANN EITLE
(86) International application number: PCT/US2003/031842
(87) International publication number: WO 2004/032934

(56) References cited:
- US-B1- 6 194 415
- P. CHOPIN ET AL: "Effects of Acute and Subchronic administration of Dexefaroxan, an alpha-2 Adrenoceptor Antagonist, on Memory Performance in Young Adult and Aged Rodents" THE JOURNAL OF PHARMACOLOGY AND EXPERIMENTAL THERAPEUTICS, vol. 301, no. 1, April 2002 (2002-04), pages 187-196, XP002272506
- P. CHOPIN ET AL: "Effects of Alpha-2 Adrenoceptor agonists and Antagonists on Circling Behaviour in Rats with Unilateral 6-Hydroxydopamine Lesions of the Nigrostriatal Pathway" THE JOURNAL OF PHARMACOLOGY AND EXPERIMENTAL THERAPEUTICS, vol. 288, no. 2, February 1999 (1999-02), pages 798-804, XP002272507
- S. TELLEZ ET AL: "Alpha2-adrenoceptor modulation of cortical acetylcholine release in vivo " NEUROSCIENCE, vol. 89, no. 4, April 1999 (1999-04), pages 1041-1050, XP002272508
- BURKE J A ET AL: "OCULAR EFFECTS OF A RELATIVELY SELECTIVE ALPHA2 AGONIST (UK-14, 304-18) IN CATS, RABBITS AND MONKEYS" CURRENT EYE RESEARCH, IRL PRESS, OXFORD, GB, vol. 5, no. 9, 1 September 1986 (1986-09-01), pages 665-676, XP000578315 ISSN: 0271-3683 cited in the application

## Description

### FIELD OF THE INVENTION

The present invention relates to the use of brimonidine or a pharmaceutically acceptable salt thereof for the manufacture of a medicament for the protection of nerve cells, particularly those of the central nervous system of mammals, from damage due to noxious insult, including glutamate toxicity and apoptosis. The use of the present invention employs the alpha 2 adrenergic receptor agonist brimonidine to prevent nerve cell damage and death, such as that observed in Parkinson's disease and Alzheimer's disease.

### BACKGROUND OF THE INVENTION

The present invention relates to pharmaceutical compositions, and particularly pharmaceutical compositions incorporating compounds which are capable of affecting alpha 2 adrenergic receptors. The present invention also relates to the use of brimonidine or a pharmaceutically acceptable salt thereof for the manufacture of a medicament for treating a wide variety of conditions and disorders, and particularly conditions and disorders associated with dysfunction of the central nervous system.

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than they bind the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into alpha 1, alpha 2, beta 1, and beta 2 subtypes.

Furthermore, it has since been recognized that each of these receptors has a number of subtypes; thus the human alpha 2 receptor can be further broken down into the alpha 2A, alpha 2B and alpha 2C receptor subtypes.

Functional differences between alpha 1 and alpha 2 receptors have been recognized, and compounds which exhibit selective binding between these two subtypes have been described.

Thus, in WO 92/00073, the ability of the R(+) enantiomer of terazosin to selectively bind to adrenergic receptors of the alpha 1 subtype was reported. The alpha 1/alpha 2 selectivity of this compound was disclosed as being significant because agonist stimulation of the alpha 2 receptors was said to inhibit secretion of epinephrine and norepinephrine, while antagonism of the alpha 2 receptor was said to increase secretion of these hormones. Thus, the use of non-selective alpha-adrenergic blockers, such as phenoxybenzamine and phentolamine, was said to be limited by their α₂ adrenergic receptor mediated induction of increased plasma catecholamine concentration and the attendant physiological sequelae (increased heart rate and smooth muscle contraction). It is significant that the selectivity of compounds termed "alpha 1 selective" or "alpha 2 selective" have traditionally been based on K_{D} data, which is limited to comparison of binding affinities to receptors, and does not compare the actual biological activities at the compared receptors.

In contrast, a method for measuring alpha receptor agonist selectivity comprises the RSAT (Receptor Selection and Amplification Technology) assay as reported in Messier et al., High Throughput Assays of Cloned Adrenergic, Muscarinic, Neurokinin And Neurotrophin Receptors In Living Mammalian Cells, Pharmacol. Toxicol. 76:308-11 (1995) and has been adapted for use with alpha 2 receptors. The assay measures a receptor-mediated loss of contact inhibition that results in selective proliferation of receptor-containing cells in a mixed population of confluent cells. The increase in cell number is assessed with an appropriate transfected marker gene such as b-galactosidase, the activity of which can be easily measured in a 96-well format. Receptors that activate the G protein, Gq, elicit this response. Alpha₂ receptors, which normally couple to Gᵢ, activate the RSAT response when coexpressed with a hybrid Gq protein that has a Gᵢ receptor recognition domain, called G_{q}/i5² . See Conklin et al., Substitution Of Three Amino Acids Switches Receptor Specificity of Gqa To That Of Gia, Nature 363:274-6. (1993).

Various alpha adrenergic receptor agonists have been reported as being useful for treating a wide variety of conditions and disorders. Thus, alpha adrenergic receptor agonists such as clonidine have been described and used as systemic and ocular hypotensive agents, as agents useful in the treatment of withdrawal from addictive behaviors such as smoking and drug abuse, and as antidysmenorrheal agents. Another alpha adrenergic receptor agonist, tizanidine, has been used for the treatment of symptoms of spasticity in multiple scelerosis patients by decreasing muscle tone. These agents have also been reported to certain analgesic activities.

These agents, while useful, have been plagued with sometimes serious side effects including sedation, cardiovascular effects such as hypotension and decreased heart rate, and dizziness that have limited their applicability for certain indications. In particular, these agents tend to have overlapping therapeutic and sedation dose response curves, such that sedative activity begins to be noticeable at the same doses as the appearance of therapeutic (e.g., hypotensive or analgesic) activity in vivo.

Compounds such as, without limitation, clonidine tizanidine, and dexmedetomidine have been characterized in the literature as "alpha 2 adrenergic receptor agonists", based largely on binding studies. See also Hieble et al., J. Med Chem. 38:3415 (September 1, 1995); Ruffolo, et al., J. Med. Chem. 38: 3681 (September 15, 1995). While it is true that these agents are alpha 2 receptor agonists, it is not generally appreciated that these agents also contain significant amounts of alpha 1 receptor agonist activity. Nor has the effect of such alpha 1 receptor activity on alpha 2 activity been generally known or appreciated.

By contrast, the compound brimonidine and its functionally similar 2-inidazolin-2-ylimino derivatives (as described below) are alpha 2 agonists which exhibit a markedly greater agonist activity towards the alpha 2 receptors than towards the alpha 1 receptor subtypes.

CNS disorders are a type of neurological disorder. Several CNS disorders can be attributed to a cholinergic deficiency, a dopaminergic deficiency, an adrenergic deficiency and/or a serotonergic deficiency. CNS disorders of relatively common occurrence include presenile dementia (early onset Alzheimer's disease), senile dementia (dementia of the Alzheimer's type), and Parkinsonism including Parkinson's disease.

The foundation of today's understanding of Alzheimer's disease is based upon the observation that certain regions of the brain of affected persons, such as the hippocampus and the cerebral cortex, showed evidence of a loss of nerve cells. Since the 1970s researchers have known that some of these dying neurons are cholinergic--that is, they communicate using the neurotransmitter acetylcholine, which is ultimately broken down by an enzyme called acetylcholinesterase. See, Jones, et al., Intern. J. Neurosci. 50:147 (1990); Perry, Br. Med. Bull. 42:63 (1986); and Sitaram, et al., Science 201:274 (1978).

Drugs that became available in the past decade, such as tacrine and donepezil, are acetylcholinesterase inhibitors. By preventing the breakdown of acetylcholine, these compounds slow the development of the early stages Alzheimer's disease. However, once cholinergic neurons degenerate fully and can no longer produce acetylcholine neurotransmitter, these drugs become useless.

Besides noting the loss of nerve cells, the brains of patients suffering from Alzheimer's disease characteristically contain clusters of proteins. These accumulations occur in two forms: those found inside neurons and those found in the intercellular space. Intracellular clusters are called neurofibrillary tangles, and appear like pairs of fibers wound around each other in a helix. Analyses have shown that tangles consist of tau protein. Tau is significant because it binds to tubulin, which is responsible for microtubule formation. The number of neurofibrillary tangles appears to correlate with the severity of the disease.

The intercellular protein clusters or plaques are composed of deposits of β-amyloid protein. The nearby neurons often appear swollen and deformed, and the amyloid plaques are usually accompanied by inflammatory microglia. The microglia, which are part of the brain's immune system, may be present in an attempt to degrade and remove damaged neurons or perhaps the plaques themselves.

It is unclear whether the neurons in or near these plaques function normally, because the density of plaques is only weakly correlated with the severity of dementia. Further, such plaques are present in most elderly people, whether they have Alzheimer's disease or not. Nevertheless, their extensive presence in the hippocampus and the cerebral cortex is specific to Alzheimer's patients, and they appear long before neurofibrillary tangles do.

β-amyloid plaques contain a 42 amino acid fragment of an integral membrane protein termed β-amyloid precursor protein (BAPP). This fragment is generated by a two-step cleavage of the BAPP protein, first by a protease termed β secretase and then by gamma secretase. The normal cleavage product of β secretase and gamma secretase is a 40 amino acid peptide, which, unlike the 42 amino acid derivative, does not appear to be involved in the initiation or progression of Alzheimer's disease.

Parkinson's disease (PD) is a debilitating neurodegenerative disease, presently of unknown etiology, characterized by tremors and muscular rigidity. A feature of the disease appears to involve the degeneration of dopaminergic neurons (i.e., which secrete dopamine), particularly in the substantia nigra and ventral tegmental regions of the midbrain. See, Rinne, et al., Brain Res. 54:167 (1991) and Clark, et al., Br. J. Pharm. 85:827 (1985). The substantia nigra is involved in the coordination of neural signals for movements and posture. The ventral tegmental area (VTA) of the midbrain contains neurons which project to sites including the prefrontal cortex, the area of the brain associated with the higher cognitative functions.

Certain attempts have been made to treat PD. One proposed treatment for PD is SINEMET®, which is a sustained-release tablet containing a mixture of carbidopa and levodopa, available from The DuPont Merck Pharmaceutical Co. Another proposed treatment for PD is ELDEPRYL®, which is a tablet containing selefiline hydrochloride, available from Somerset Pharmaceuticals, Inc. Another proposed treatment for PD is PARLODEL®, which is a tablet containing bromocriptine mesylate, available from Sandoz Pharmaceuticals Corporation. Another method for treating PD and a variety of other neurodegenerative diseases through melanin therapy has been proposed in U.S. Pat. No. 5,210,076 to Berliner et al. However, none of these treatments appear to protect neurons from cell death.

### SUMMARY OF THE INVENTION

While it has been known that brimonidine and its derivatives are able to provide neuroprotective activity to optic or retinal cells and the nerve cells of the spine when applied topically or injected at the site of nerve damage, it has heretofore not been thought that such agents would be effective agents for the treatment of neurodegenerative conditions of the brain, such as Alzheimer's disease and Parkinson's disease, in part due to the blood brain barrier, and in part due to the significant sedative activity that accompanies the administration of other known alpha 2 adrenergic receptor agonists such as clonidine, tizanidine and dexmetatomidine. Thus, such sedative activity upon systemic administration of such agents at therapeutic doses have severely limited their usefulness as a practical matter as non-topical or systemic agents.

The present Applicants have surprisingly discovered that brimonidine and its derivatives are able, when administered systemically, to provide neuroprotection to the nerve cells of the brain. Brimonidine and its derivatives have a dramatically broader therapeutic window between their neuroprotective activity and their sedative activity than most previously characterized alpha adrenergic agonists.

Brimonidine and its therapeutic value was disclosed in Danielewicz, et al. in U.S. Pat. Nos. 3,890,319 and 4,029, 792. These patents disclose brimonidine as a regulators of the cardiovascular system which have the following formula: 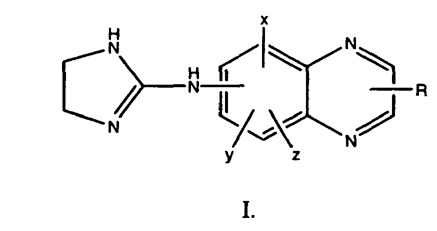 where the 2-imidazolin-2-ylamino group may be in any of the 5-, 6-, 7- or 8-position of the quinoxaline nucleus; x, y and z may be in any of the remaining 5-, 6-, 7- or 8-positions and may be selected from hydrogen, halogen, C₁₋₅ alkyl, C₁₋₅ alkoxy or trifluoromethyl; and R is an optional substituent in either the 2- or 3-position of the quinoxaline nucleus and may be hydrogen, C₁₋₅ alkyl or C₁₋₅ alkoxy. The presently useful compounds may be prepared in accordance with the procedures described in Pat. Nos. 3,890,319 and 4,029,792, hereby incorporated by reference herein.

In *Ocular Effects of a Relatively Selective Alpha-2 Agonist (*UK-14,304-18*) in Cats, Rabbits and Monkeys,* J. A. Burke, et al., Current Eye Rsrch., 5, (9), pp. 665-676 (1986) the quinoxaline derivative shown below and having the generic name brimonidine was shown to be effective in reducing intraocular pressure in rabbits, cats and monkeys. Compounds in this study were administered topically to the corneas of the study animals.

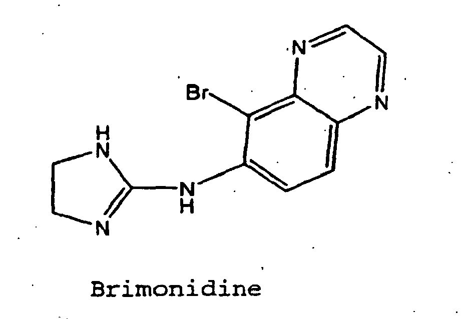

It is known that the alpha 2 receptor agonist brimonidine can protect retinal neural cells, including photoreceptors and retinal ganglion cells, from damage in conditions such as glaucoma, retinitis pigmentosa, and age-related macular degeneration when administered topically or systemically. See U.S. Patent 6,194,415.

In a first aspect the present invention is directed to the use of brimonidine or a pharmaceutically acceptable salt thereof for the manufacture of a medicament for treating a neurodegenerative condition of the brain comprising administering to the brain of a mammal in need thereof a therapeutically effective amount of brimonidine.

Brimonidine has less alpha 1 receptor activity than the other well known alpha 2 receptor agonists. Although these agonists are normally thought of as alpha 2 "selective" agonists, in fact all of these compounds have greater activity at the alpha 1A receptor than at at least one alphga 2 receptor subtype. By contrast brimonidine has at least 5.5-fold greater activity at each alpha 2 recpetor subtype that at the alpha 1A receptor. The data presented below was collected using the RSAT assay described above.

| **Compound** | **EC50** | | | |
|---|---|---|---|---|
| | 1A | 2A | 2B | 2C |
| **Clonidine** | 13 | 13 | 22 | 65 |
| **Tizanidine** | 351 | 207 | 127 | 1693 |
| **Dexmedetomidine** | 11 | 2 | 2 | 1 |
| **Brimonidine** | 850 | 17 | 60 | 33 |

The present inventors have also noticed that brimonidine also has a wider therapeutic window - that is, a greater difference in the concentration necessary to provide a therapeutic effect mediated by the alpha 2 receptor, as compared to that necessary to cause sedation, than other compounds having greater alpha 1 receptor activity.

Thus, both the therapeutic and sedative effects of Tizanidine and clonidine are seen beginning at concentrations of about 100 micrograms/kg when applied IP. By contrast, while the therapeutic effects of brimonidine can begin to be measured at 10 micrograms/kg, the sedative effect of brimonidine first becomes noticeable at about 30 micrograms/kg. Brimonidine can thus be used systemically to provide neuroprotective activity with less concern of oversedating the patient.

Efficacy of a given receptor or receptor subtype in accordance with the-present invention is determined using the RSAT assay procedure described above.

While Applicants do not wish to be limited by theory, it is thought that lessening the stimulation of the alpha 1 receptor(s) serves to cause a diminution of the EC₅₀ of brimonidine (leading to a therapeutic effect at a lower concentration of drug) relative to to similar compounds having a greater amount of alpha 1 receptor activity, with no change in the sedation dose-response curve.

In another aspect, the present invention is directed to the use of brimonidine as a pharmaceutically acceptable salt thereof for the manufacture of a medicament for preventing death or degeneration of neural cells projecting to or from a region of the brain including the locus ceruleus comprising administering a thereapeutic amount of brimonidine to said cells.

This new use is particularly effective when administered as a prophylactic treatment, i.e. before damage to the nerve has taken place, or before long-term progression of the disease state, such as Alzheimer's or Parkinson's disease, has taken place. Without wishing to be held to a particular theory regarding the role that the compounds of the present invention play in neuroprotection, Applicants hypothesize that brimonidine, when used in accordance with the use described herein may stimulate the production of certain factors of the bcl-2 family; the increased expression of such factors has been measured by the increased expression of mRNA encoding their production; these factors (bcl-2 and bcl-XL can suppress the apoptotic program. These factors can counterbalance the presence or induction of bcl-2 apoptosis factors such as bad and bax which may be produced as a result of noxious provocations to the nerve cells. Thus, it is further contemplated that the compounds of the present invention which provide cell survival signals to the nerve can advantageously be used in combination with compounds that inhibit cell death. Such cell death inhibiting compounds include NMDA antagonists, especially memantine, which block excitotoxic effects of excess glutamate; nitric oxide synthetase inhibitors; free-radical scavengers and calcium channel blockers.

Any suitable method of administering brimonidine to the brain of the mammal to be treated may be used. In all the methods, the preferred mammal is a human. The particular method of administration chosen is preferably one which allows brimonidine to have the desired therapeutic effect in an effective manner, e.g., low effective concentration and low incidence of side effects.

Administration of brimonidine in a manner consistent with the methods of this invention can include, but are not limited to, oral, parenteral, intravenous, subcutaneous and other modes of systemic administration. The compounds are administered in a therapeutically effective amount either alone or in combination with a suitable pharmaceutically acceptable carrier or excipient.

Depending on the intended mode of administration, a therapeutic amount of brimonidine may be incorporated in any pharmaceutically acceptable dosage form, such as for example, tablets, suppositories, pills, capsules, powders, liquids, solutions, infusions, suspensions, emulsions, aerosols or the like, preferably dosage forms suitable for single administration of precise dosages, or sustained release dosage forms for continuous controlled administration. Preferably, the dosage form will include a pharmaceutically acceptable excipient and the presently useful compound or compounds and, in addition, may contain other medicinal agents, pharmaceutical agents, carriers, adjutants, etc.

For solid dosage forms, non-toxic solid carriers include, but are not limited to, pharmaceutical grades of mannitol, lactose, starch, magnesium stearate, sodium saccharin, the polyalkylene glycols, talcum, cellulose, glucose, sucrose and magnesium carbonate. An example of a solid dosage form for carrying out the invention is a suppository containing propylene glycol as the carrier.

Liquid pharmaceutically administrable dosage forms can, for example, comprise a solution or suspension of one or more of the presently useful compounds and optional pharmaceutical adjutants in a carrier, such as for example, water, saline, aqueous dextrose, glycerol, ethanol and the like, to thereby form a solution or suspension. If desired, the pharmaceutical composition to be administered may also contain minor amounts of nontoxic auxiliary substances such as wetting or emulsifying agents, pH buffering agents and the like. Typical examples of such auxiliary agents are sodium acetate, sorbitan monolaurate, triethanolamine, sodium acetate, triethanolamine oleate, etc. Actual methods of preparing such dosage forms are known, or will be apparent, to those skilled in this art; for example, see Remington's Pharmaceutical Sciences, Mack Publishing Company, Easton, Pa., 16th Edition, 1980, incorporated by reference herein. The composition of the formulation to be administered, in any event, contains a quantity of one or more of the presently useful compounds in an amount effective to provide the desired therapeutic effect.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions or infusions. Suitable excipients are, for example, water, saline, dextrose, glycerol, ethanol and the like. In addition, if desired, the injectable or infusible pharmaceutical compositions to be administered may also contain minor amounts of non-toxic auxiliary substances such as wetting or emulsifying agents, pH buffering agents and the like.

The amount of brimonidine administered is, of course, dependent on the preceise therapeutic effect or effects desired, on the specific mammal being treated, on the severity and nature of the mammal's condition, on the manner of administration, on the potency and pharmacodynamics of the particular compound or compounds employed, and on the judgment of the prescribing physician. Generally, the therapeutically effective dosage is preferably in the range of about 0.5 or about 1 to about 100 mg/kg/day.

### DETAILED DESCRIPTION OF THE INVENTION

In one aspect the present invention is drawn to the use of brimonidine or a pharmaceutically acceptable salt thereof for the manufacturing of a medicament for treating a neurodegenerative condition of the brain comprising administering to the brain of a mammal in need thereof a therapeutically effective amount of brimonidine or a pharmaceutically active salt thereof.

In connection thereto the Applicants have discovered that brimonidine has an unexpectedly increased efficacy in protecting the neural cells of the brain against damage and death, including apoptosis, over compounds that have alpha 1 receptor agonist activity. While not wishing to be limited by theory, Applicants believe that stimulation of the alpha 1 adrenergic receptor results in interference with the neuroprotective activity provided by the alpha 2 agonist activity such that any sedative effect that such agents as clonidine or tizanidine may have has an EC50 similar, or within about a three-fold range of the neuroprotective activity for such compound. Thus, any neuroprotective activity provided by non-selective agents such as clonidine, tizanidine and dexmetatomidine is seen at concentrations that will tend to sedate or be toxic to the patient.

In part for this reason, alpha adrenergic agents have not generally been used as neuroprotective agents in the past, except in topical or local applications (such as ophthalmic applications) in which the agent is not generally administered systemically.

While not wishing to be limited by theory, the present applicants believe that most or the entire neuroprotective efficacy of brimonidine is provided by stimulation of the alpha 2B and/or alpha 2C receptors. The brain has not generally been thought to be rich in alpha 2B or 2C receptors. However, the Applicants have found that the use of brimonidine or a pharmaceutically acceptable salt thereof for the manufacture of a medicament according to the present invention can provide neuroprotective effects to neurons projecting from or to the locus coruleus, site of the initial widespread damage noted in Alzheimer's disease. Thus, use of brimonidine in a manner consistent with the present disclosure will be useful in the treatment of neural damage in conditions such as Alzheimer's disease and Parkinson's disease.

### The Link Between Glaucoma and Alzheimer's Disease

While not wishing to be limited by theory, the Applicants offer the following as a hypothesis why brimonidine is an effective neuroprotector in brain neurodegenerative conditions such as Alzheimer's and Parkinson's disease.

Recent studies have suggested glaucomatous loss of retinal ganglion cells RGCs) and their axons in Alzheimer's disease (AD). Amyloid β peptides and phosphorylated tau protein have been implicated in the selective regional neuronal loss and protein accumulations characteristic of AD. Similar protein accumulations are not present on glaucomatous RGCs. Neurons die in both AD and glaucoma by apoptosis, although the signaling pathways for neuronal degradation appear to differ in the two diseases. AD features a loss of locus coeruleus noradrenergic neurons, which send axon terminals to the brain regions suffering neuronal apoptosis and results in regional reductions in noradrenaline (NA) and neuronal α2 adrenergic receptor levels. Activation of α2 adrenergic receptors reduces neuronal apoptosis through a protein kinase B (Akt) dependent signaling pathway. Loss of NA innervation may facilitate neuronal apoptosis in both AD and glaucoma. α2 adrenergic receptor agonists offer the potential to slow the neuronal loss in both diseases by compensating for lost NA innervation.

Neurodegenerative diseases most often disable rather than kill their victims. The progressive nature and prolonged time course of neurodegeneration imposes a marked economic burden on our Society, in large part due the numbers of care givers necessary to aid affected persons. Glaucoma meets the criteria for a typical neurodegenerative disease. Retinal ganglion cells (RGCs) die gradually and progressively in the disease, often in association with increased intra-ocular pressure (IOP). Glaucomatous neuronal death may not be limited to the retina since neurons in the lateral geniculate nucleus, and even the visual cortex, seem to be lost. The pathogenesis of glaucomatous neurodegeneration is not understood with certainty. A variety of evidence points to impaired RGC axonal protein transport due to IOP-induced compression of optic nerve axons at the lamina cribosa. Trophic factors, like brain derived neurotrophic factor (BDNF), are retrogradely transported from RGC axonal terminals to the cell bodies of the neurons and that transport of trophic factors is essential to RGC survival. RGC axon compression or crush can reduce trophic factor levels causing RGC death by trophic insufficiency. Other evidence has implicated local ischemia-hypoxia, over activation of glutamate receptors, excessive generation of oxidative radicals by nitric oxide or activation of immune-related receptors as possible contributors to glaucomatous neuronal loss.

### A Relationship Between Glaucoma And Alzheimer's Disease (AD)

Similar to glaucoma, trophic insufficiency, oxidative radical damage, hypoxia and immune-related mechanisms have all been cited as possible contributors to neuronal loss in Alzheimer's disease (AD). Cortical, hippocampal, septal, thalamic and brainstem neurons are principally lost in AD but RGCs may be lost as well. Histological examinations of postmortem retinae and optic nerves have suggested that RGCs and their axons atrophy and die in AD. In several studies, optic nerve axonal loss was extensive since the average numbers of axons in AD optic nerves (68/1000 µm²) were less than half those found in age matched controls (116 /1000 µm²). Size distributions of optic nerve axons suggested that large axons were lost preferentially in AD similar to that reported for idiopathic glaucoma. Other studies of postmortem AD optic nerve have failed to find evidence for RGC axonal degeneration. The different findings of the postmortem studies are difficult to evaluate since small numbers of AD patients and age matched controls (n = 7 to 10) were examined in each of the studies; the stage of AD progression was not well controlled and likely varied between studies; and the histological and counting methods differed from one study to another. Measurements of retinal nerve fiber layer thickness, nerve head pallor and cupping etc. using *in vivo* techniques in AD patients have also differed extensively in different studies. They have ranged from finding no evidence of glaucomatous changes to finding well defined glaucomatous changes, whose extent appeared to correlate with the level of cognitive decline in each of the patients. The most compelling evidence for an association between AD and glaucoma has been provided by the extensive study of Bayer et al. They showed that about 26 % of patients with AD (n = 112) have retinal glaucomatous changes, particularly visual field loss and optic nerve cupping, while only 5 % of age matched controls (n = 116) had glaucomatous changes.

Neurofibrillary tangles (NFTs) and amyloid plaques are the pathological hallmarks of neurodegeneration in AD. Amyloid precursor protein (APP) is a membrane protein that can be cleaved to secreted APP (sAPPα) or alternatively to Aβ1-40 peptide (Aβ1-40) by α-secretase and β-amyloid cleavage enzyme (BACE) respectively. sAPPα is the predominant APP derivative in most cell types. A larger form of Aβ peptide, Aβ1-42, aggregates readily and is the major component of the core of amyloid plaques. Either defective APP processing or degradation may be responsible for high levels of Aβ1-42 peptide in AD. Aβ1-42 peptide is toxic to cultured cells and may be to be responsible for AD neuronal loss. Genetic studies have identified mutations in APP as possible triggers for the pathogenesis of AD. Other genes such as those for presenilins 1 and 2 (PS1/2) and apolipoprotein E (APOE) may contribute to AD by increasing Aβ levels.

NFTs are primarily comprised of tau, a microtubule associated protein. In AD, tau becomes highly phosphorylated and aggregates into filaments in neuronal cell bodies. Phosphorylated tau appears to have a decreased capacity to bind to microtubules and aggregates with neurofilament proteins to form NFTs. Phosphorylated tau likely plays a critical role in Aβ toxicity and Aβ1-42 peptide may promote tau aggregation and hyperphosphorylation by tau protein kinase II. The two proteins, Aβ1-42 peptide and phosphorylated tau, may therefore facilitate the conformations of the other that promotes AD neuronal toxicity.

Some increases in tau, APP, and Aβ peptide immunoreaction have been reported in the RGC layer and the pigment epithelium of aged human retina as well as retina from persons with retinitis pigmentosa and age-related macular degeneration. Those increases in levels of immunoreaction were not accompanied by NFTs or amyloid plaques. Similarly, increases in Aβ peptide immunoreaction in RGCs have been reported for a rat ocular hypertensive model using immunocytochemistry. Whole retinal immunoblots for the same hypertensive model have suggested that the increased Aβ peptide immunoreaction is accompanied by decreases in full-length APP and increases in Aβ-containing fragments. It has been hypothesized that RGC axon compression in glaucoma may facilitate neurofilament protein abnormalities and Aβ peptide induced neuronal damage. Despite the evidence for APP, Aβ peptide and tau abnormalities in the rat glaucoma model and in several non glaucomatous retinal conditions, the optic nerve axonal loss and RGC degeneration in AD has not been reported to include either the NFTs or amyloid plaques. Accordingly, it is uncertain whether RGC degeneration in AD results from the APP and tau abnormalities thought to underlie central nervous system (CNS) neuronal loss in AD.

### Neurons May Die In Glaucoma And AD By Apoptosis.

A death process called apoptosis appears to contribute to neuronal loss in both glaucoma and AD. Apoptosis includes a process of gradual cellular degradation that features nuclear and cellular shrinkage combined with cleavage of nucleic acids and cytoskeletal proteins by endonucleases and proteases. Although most investigations have supported a role apoptosis in the neuronal loss found in glaucoma or AD, others have questioned the involvement of apoptosis in the diseases. The differing views have been largely based on the morphological criteria deemed necessary to define apoptosis and/or the interpretation of evidence for nuclear DNA cleavage obtained using terminal deoxynucleotidyl transferase-mediated deoxyuridine triphosphate nick-end labeling (TUNEL). Apoptotic nuclear degradation can be difficult to detect on neuropathological examination due to the shrinkage, degradation and phagocytosis of affected neurons. The degradation process has an apparent life of less than 24 hours in culture and a matter of days in intact nervous tissue. Accordingly at any single time of examination, only a small percentage of neurons can be expected to show evidence of apoptotic degradation in diseases with a time course of years.

DNA gel electrophoresis or pulse field electrophoresis can be used to detect nuclear DNA cleavage as a marker of apoptotic degradation. The procedures examine DNA taken from tissue homogenates. They require that the homogenates include that 10⁵ or more cells, which are in the stage of apoptotic degradation, before nuclear DNA cleavage can be detected. Given the prolonged time course of AD or glaucoma, only a small number of neurons would be expected to undergo nuclear degradation over the course of any day with the result that insufficient fragmented DNA is present in homogenates from an AD brain or a glaucomatous retina. The labeling of cut 3' DNA ends with d-UTP attached to a fluorochrome has been used to reveal nuclear DNA cleavage *in situ* in tissue sections or whole mounts from postmortem AD brain or glaucomatous retinae. TUNEL positive RGC nuclei have been found in human glaucomatous retinae and in the retinae of glaucoma animal models. The small proportion of TUNEL positive RGC nuclei has seemed appropriate for the gradual loss of RGC in glaucoma. The finding of TUNEL positive neurons has varied in AD as some studies have found increased numbers of TUNEL positive neurons in brain regions affected by the disease, while others have failed to detect any increases in TUNEL positive neurons in AD brain compared to those from age-matched controls. Furthermore, several studies found inappropriately high counts of TUNEL positive neurons in AD brain, for example 25% of neurons were reported to be TUNEL positive in some cortical regions.

The variation in the TUNEL findings in AD probably result from methodological difficulties known to affect the labeling technique, particularly when it is used in postmortem nervous tissue 1) reactive oxygen species damage to DNA, independently of DNA cleavage by endonucleases, can be detected by TUNEL; 2) delayed tissue fixation, over fixation or prolonged fixation can induce TUNEL labeling; 3) cells entering mitosis can be TUNEL labeled; 4) different endonucleases can cut DNA differently, which can dramatically affect TUNEL labeling efficiency; and 5) variations in divalent cations concentrations, like Mg²⁺ or Co²⁺, markedly affect TUNEL labeling. Accordingly, if TUNEL labeling is used by itself to establish apoptotic degradation, the results should be interpreted with caution whether they are positive or negative. TUNEL labeling can be valuable in discerning apoptotic degradation if it is used jointly with other markers of the degeneration, particularly DNA-binding dyes that reveal chromatin condensation or immunocytochemistry for degradation signaling proteins.

### Apoptotic Degradation Signaling In Glaucoma And AD

Apoptotic signaling processes can be considered to involve three stages: 1) a pre-mitochondrial stage; 2) a mitochondrial stage and 3) a post-mitochondrial or degradation stage. As well as nuclear DNA cleavage, apoptotic degradation involving the nucleus can include: 1) the separation of histones and lamins from nuclear DNA; 2) the condensation or compaction of the fragmented DNA; and 3) the formation of membrane wrapped sub-nuclear bodies containing fragmented and condensed DNA. The extent, pattern and time course of apoptotic nuclear degradation been shown to differ for different cellular phenotypes and for different insults to the cells. We have shown variation in the patterns of apoptotic nuclear changes using multiple degradation markers in RGCs in postmortem glaucomatous eyes or in glaucoma animal models; MPTP-exposed murine nigral neurons, hypoxic neurons in the porcine hippocampus, and nigral neuromelanin containing neurons in PD postmortem brain. Accordingly, it can be confusing to label one form of nuclear degradation as apoptotic and others as non-apoptotic based on nuclear morphology.

Apoptotic cellular degradation is now known to depend on a number of interacting signaling pathways. Different insults and/or cellular phenotypes can activate different degradation signaling, which can be reflected.in differences in the morphology of nuclear changes, notably those for chromatin condensation and/or DNA fragmentation. The caspase family of proteases plays fundamental roles in many apoptosis signaling pathways. More than a dozen caspases have now been characterized in mammals. Caspases are expressed as inactive pro-enzymes, which are proteolytically activated and can cleave other caspases, cytoskeletal proteins, nuclear proteins, or anti-apoptotic signaling proteins. Four different signaling pathways are known to contribute to nuclear chromatin condensation and DNA fragmentation. Two of the pathways are caspase dependent: 1) the cytochrome C, apoptotic protease-activating factor 1 (Apaf-1) and pro-caspase 9 pathway; and 2) the second mitochondrial derived activator of caspases (SMAC)/direct IAP binding protein (Diablo) pathway. The other two pathways are caspase independent: 1) the apoptosis initiation factor (AIF) pathway and 2) the endonuclease G pathway.

It was first discovered that cytochrome C released from mitochondria interacts with Apaf-1 and dATP to convert of pro-caspase 9 to activated caspase 9. Activated caspase 9 is also released from mitochondria. Activated caspase 9 performs the key step in a number of forms of apoptotic degradation by converting pro-caspase 3 to activated caspase 3. Activated caspase 3 cleaves inhibitor of caspase-activated DNase (ICAD) to caspase-activated DNase (CAD), which fragments nuclear DNA. Activated caspase 3 also can signal for other aspects of apoptotic cellular degradation including nuclear chromatin condensation through protease-activation of acinus, actin cytoskeletal digestion by the protease gelosin and nuclear lamin cleavage through caspase 6.

Caspase 3 activation has been reported in glaucomatous RGCs. Although several studies found neuronal caspase 3 activation in AD, others have failed to find evidence for participation of a cytochrome C - caspase 3- CAD pathway in neuronal DNA fragmentation in AD. Caspases, like caspase 3, can be inhibited by one or more members of a family of constitutively active proteins, the inhibitors of apoptosis (IAPs). IAPs bind to and inactivate the caspases and thereby can prevent or reduce nuclear degradation by the cytochrome C - caspase 3 - CAD pathway. In turn, SMAC/Diablo, released from mitochondria, can bind and inactivate the IAPs, thereby allowing caspases 9, 3 and 6 to signal for apoptotic degradation. Although IAPs have been implicated in the AD-like neurodegeneration in Down syndrome and in rat RGCs after axotomy, it is not known whether IAPs or SMAC/Diablo affect the caspase 3 degradation pathway in either AD or glaucoma.

In some forms of apoptosis, a soluble flavoprotein, AIF, is released from the intermembrane space of mitochondria and translocates to nuclei where it induces DNA fragmentation and also contributes to chromatin condensation. AIF can be released from mitochondria independently of cytochrome C and pro-caspase 9. The basis for selective release of AIF from mitochondria is not known. Microinjection of cells with recombinant AIF only causes chromatin condensation in the outer portion of the nucleus, whereas microinjection with activated caspase 3 or its downstream target CAD causes whole nuclear chromatin condensation. In some other forms of apoptosis, AIF induces cytochrome C release from mitochondria. Hence nuclear DNA cleavage and chromatin condensation can be induced with different sub-nuclear distributions by caspase 3 alone, AIF alone and/or by AIF followed by caspase 3. A mitochondrial DNase, endonuclease G, which normally functions to cleave mitochondrial DNA, can also be released from mitochondria in some forms of apoptosis and serve to directly induce nuclear DNA fragmentation. Nuclear DNA fragmentation caused by endonuclease G is independent of either caspase or CAD activity. Endonuclease G can be released from mitochondria by BID and is a feature of apoptosis induced by FAS ligand (see below). Little is known of the sub-nuclear pattern of DNA fragmentation induced by endonuclease G, but it likely adds a further permutation to the possible patterns of apoptotic nuclear degradation involving caspase 3 and/or AIF.

Given our growing understanding of the signaling pathways for degradation in apoptosis, it is not surprising that a number of different morphological forms of nuclear degradation have been observed. Based on our current information, it seems that glaucomatous RGC apoptotic degradation involves, at least in part, a caspase 3 signaling pathway while AD apoptotic degradation may be either caspase dependent or caspase independent and therefore possibly reflect AIF and/or endonuclease G signaling as well as caspase 3 activation.

### Mitochondrial Apoptosis Signaling In Glaucoma And AD

How do neurons decide to activate the signaling pathways for apoptotic degradation? Changes in mitochondrial membrane permeability with the release of factors that signal for apoptotic degradation constitutes a critical decisional step in many apoptosis signaling pathways. One or more of the signaling factors for apoptotic degradation (cytochrome C, pro-caspase 9, AIF, SMAC/Diablo or endonuclease G) are released in the intermembranous space that separates the inner and outer mitochondrial membranes or are released from the mitochondrial matrix. Two mechanisms have been suggested to cause increased outer membrane permeability: 1) the formation of pores or the opening of existing pores in the outer membrane (reviewed in; and 2) opening of a multi-protein megapore, the permeability transition pore complex (PTPC), which spans the inner and outer mitochondrial membranes.

As schematized in figure 1, BAX, in association with its pro-apoptotic relative BAK, may contribute to PTPC opening or to outer membrane permealization, although the basis for BAX-induced changes in outer mitochondrial membrane permeability are not well understood. BAX oligomers can insert into planar phospholipoid bilayer membranes and promote dissolution of the membranes. In some apoptosis models, BID can induce BAX oligomerization which can induce the release of cytochrome C or SMAC/Diablo from liposomes or mitochondria. BID can induce BAX insertion into the outer mitochondrial membrane or cause BAX to bind to the PTPC. In other forms of apoptosis, BID may not be required for BAX accumulation in mitochondria.

Opening of the PTPC may cause osmotic shifts across the inner mitochondrial membrane with consequent mitochondrial matrix swelling and rupture of the outer mitochondrial membrane, which may allow the release of the apoptotic degradation factors from mitochondria. Agents like cyclosporin A, which promote PTPC closure, reduce cytochrome C release and apoptosis in some forms of apoptosis. BAX has been shown to bind to either the adenine nucleotide translocator (ANT) or the voltage dependent anion channel (VDAC) of the PTPC. BAX binding to either the ANT or VDAC markedly increases the conductance of isolated membranes. Accumulating data suggests that either ANT or VDAC may increase inner mitochondrial permeability in different forms of apoptosis. Cytochrome C may be stored in mitochondrial cristae formed from the inner membrane, which fuse with the outer membrane and allow the release cytochrome C through the PTPC. Accordingly, opening of the PTPC together with the formation of outer membrane pores may be responsible for cytochrome C release.

Immunocytochemistry of postmortem glaucomatous retinae have shown increased BAX in a small proportion of RGCs, which appears similar to that found in a rat ocular hypertension model. Increases in inner mitochondrial membrane permeability induced by BAX result in decreases in mitochondrial membrane potential and mitochondrial membrane potential has been shown to decrease in RGCs in a rat ocular hypertension model. Studies of postmortem AD brain have either shown increased BAX in a proportion of neurons or no difference in neuronal BAX levels relative to those in control brains. One study reported an increase in BAK without an increase in BAX. Accordingly, it is uncertain whether BAX dependent increases in mitochondrial membrane permeability contribute to apoptosis in AD. There may be several forms of AD - one which involves increased BAX and activated caspase 3 (see above) and a second which is independent of increases in mitochondrial membrane permeability.

### Pre-Mitochondrial Apoptosis Signaling In Glaucoma And AD.

Figure 1 presents a schematic for the possible pre-mitochondrial and mitochondrial apoptosis signaling that contribute to neuronal loss in glaucoma and AD. Two different pathways shown in figure 1 have been proposed as contributors to glaucomatous loss of RGCs: 1) a p53-glyceraldehyde-3-phosphate dehydrogenase (GAPDH) - BAX pathway; and 2) a FAS or TNF receptor - FADD - caspase 8 - BAX pathway. Similarly, both p53 and Fas or TNF signaling have been implicated in AD.

The tumor suppressor protein, p53, has been implicated in numerous forms of apoptosis and can signal for apoptosis by either transcriptional or post translational mechanisms. p53 induces transcriptionally-mediated increases in GAPDH and BAX in some forms of apoptosis. GAPDH is a multifunction protein that is best known as a glycolytic enzyme but also functions as an apoptosis signaling protein. Studies with antisense oligonucleotides showed that GAPDH can be essential to the progression of apoptosis initiated by a variety of different insults to neuronal cells. GAPDH appears to decrease the transcription of the anti-apoptotic proteins BCL-2 and BCL-X_{L}. BCL-2 and BCL-X_{L} oppose the increased mitochondrial membrane permeability and apoptotic degradation factor release induced by BAX and thereby can reduce apoptotic degradation.

The tumor necrosis factor (TNF) receptor super family, including TNF receptor and FAS receptor, cause apoptosis through caspase dependent pathways that do not involve changes in transcription. The receptors are linked to adapter proteins like FADD that activate caspases, particularly caspase 8, which as illustrated in figure 1, can activate BAX dependent increases in mitochondrial membrane permeability or can bypass mitochondria and directly induce apoptotic degradation. FAS, and possibly TNF, induced apoptosis may be linked to p53 induced apoptosis through jun-n-terminal kinase (JNK) activation (schematically shown in figure 1). It also has been shown that the FAS receptor can be up-regulated by p53 following some cellular lesions, particularly those induced by DNA-damaging agents. The p53 induced up-regulation can induce apoptosis through a FAS/FAS ligand-dependent pathway and p53 and immune/cytokine pathways.

### Noradrenaline In AD and Glaucomatous Neuronal Apoptosis.

Noradrenergic neurons in the locus coeruleus (LC) are lost early and extensively in AD and show NFT formation typical of the disease. Cephalad projecting axons of LC neurons form dense terminal arborations in the forebrain regions that suffer prominent neuronal loss in AD, including the cortex, septum, hippocampus and thalamus. In keeping with the loss of the noradrenaline (NA) containing LC neurons, those regions suffer marked reductions in NA. concentrations in AD. The LC terminals contain tyrosine hydroxylase (TH), which catalyzes the conversion of tyrosine to dopa and dopamine-β-hydroxylase (DBH), which converts dopamine to NA. Immunocytochemistry has shown marked decreases in TH and DBH immunopositive terminals in degenerating regions like the cortex and hippocampus in AD. It was initially proposed that the principal effect of noradrenergic LC terminal loss in AD resulted from loss of NA activation of adrenergic receptors. LC terminals innervate brain microvessels and capillaries through α-adrenergic receptors and increase their sensitivity with a possible decrease in regional blood flow.

Now it is known that the noradrenergic terminals can also end on or near to neurons and release noradrenaline, which activates α-adrenergic or α-adrenergic receptors on the neurons. α2-adrenergic receptors have been demonstrated on a wide variety of different neurons and/or their terminals, including sympathetic gangliar, spinal, brainstem, septal, hippocampal and cortical neurons. α-adrenergic receptors are widely but unevenly distributed in brain. They are abundant in the cerebral cortex, hippocampus, and various hypothalamic nuclei but are largely absent from thalamic (excepting in the lateral and medial geniculate nuclei) and septal nuclei.

α2-adrenergic receptors may be particularly relevant to AD. α2-adrenergic receptor binding is decreased in the cortex and hippocampus of AD postmortem brain; the loss of LC neurons and their noradrenergic terminals is probably accompanied by reductions in some α2-adrenergic receptors on the neurons that they innervate or that neurons with these α2-adrenergic receptors are lost in AD . Pharmacological activation of α2-adrenergic receptors has been found to improve human cognition and to reduce central neuronal loss after exposure to insults like ischemia.

α2-adrenergic receptors can mediate a number of different intracellular actions through G-protein dependent signaling pathways or signaling mechanisms that are G-protein independent. In relation to apoptosis, activation of the receptors can induce the phosphorylation of protein kinase B (Akt) through a pathway that depends on phosphatidylinositol-3- kinase (PI3-Kinase). Phosphorylated Akt mediates anti-apoptosis through a number of transcriptionally-dependent and post translational signaling pathways. A principal action of phosphorylated Akt lies in preventing or reducing the increases in mitochondrial membrane permeability, which are responsible for some forms of apoptotic degradation. For example, phosphorylated Akt maintains or increases the synthesis of BCL-2 and BCL-X_{L} and prevents BAD from inactivating those proteins through the formation of BCL-2/BAD or BCL-X_{L/}BAD heterodimers. BCL-2 and BCL-X_{L} oppose the increased mitochondrial membrane permeability caused by actions of BAX, BAK and BID and thereby prevent or reduce the release of mitochondrial factors that signal for apoptotic degradation. We thus believe that noradrenaline, 2-adrenergic receptor agonists and BDNF may therefore reduce apoptosis in neurons expressing α2-adrenergic receptors or Trk B receptors (the neurotrophic receptors for BDNF) and to protect neurons in AD from apoptosis. It might also be argued that a reduction of NA release due to the loss LC neurons in AD may increase the vulnerability of neurons in the cortex; septum, hippocampus and thalamus to apoptosis caused by the A peptide and/or phosphorylated tau mechanisms considered to contribute to AD pathogenesis.

While not wishing to be limited by theory, we further hypothesize that a similar loss or reduction of NA-mediated anti-apoptosis for RGCs may explain the loss of RGCs in AD and the high incidence of glaucoma in the disease. Adrenergic α-receptor agonists like B-HT920 or UK14304 (brimonidine) or α1-antagonists like betaxolol, metropolol or timolol have been used to reduce IOP in glaucoma by decreasing the production or by increasing outflow of aqueous humor, at least in part, by acting on the ciliary epithelium. Apart from adrenergic receptors in the ciliary epithelium, retinal tissue has been found to contain β2, α1 and α2 adrenergic receptors.

α2-adrenergic receptor agonists like brimonidine or clonidine have reduced RGC loss after retinal ischemia, optic nerve crush or increased IOP. Similarly, the α1-antagonist, betaxolol has been reported to reduce RGC loss after retinal ischemia or excitotoxin exposure. Recent studies in retinal culture have similarly shown that brimonidine and betaxolol reduce RGC loss caused by excitotoxins. Accordingly, activation of α2-adrenergic receptors, but not blockade of α-adrenergic receptors, can reduce the death of RGCs. Research in our laboratories has shown that α2-adrenergic receptor agonists can reduce apoptosis in cultured neuron-like cells or in primary neuronal cultures caused by trophic withdrawal, kinase inhibitors, mitochondrial toxins and excitotoxins and that the anti-apoptosis can be competitively blocked by α2-adrenergic receptor antagonists. The anti-apoptosis depends on the maintenance of mitochondrial membrane impermeability in association with increased levels of BCL-2 and phosphorylated Akt.

How might central neurons and RGCs share common apoptotic mechanisms in AD? Although the role of NA in the retina has been controversial, DBH immunocytochemistry has provided evidence for NA containing axons in the inner plexiform and ganglion cell layers of bovine, monkey and human retina, suggesting that NA fibers terminate on or near to RGC cell bodies or dendrites. NA has been demonstrated in the inner plexiform and inner nuclear layers in the bovine retina. α2-adrenergic receptor binding has been demonstrated in dissociated bovine or monkey retina. Together those findings indicate that the inner retina contains noradrenergic terminals that release NA in the region of α2-adrenergic receptors on RGCs. The source of NA containing axon terminals in the inner retina remains unknown. NA containing cell bodies have not been found in the retina, which suggests that extra-retinal neurons send noradrenergic axons into the retina. Conceivably, NA containing axons could reach the retina on the surface of blood vessels or via the optic nerve. DBH immunoreactive axons have been demonstrated in the bovine optic nerve suggesting that NA containing axons may reach the retina via the optic nerve. It will be important to determine whether NA containing neurons in the LC, the hypothalamus or sympathetic ganglia send the NA containing axons to the retina, particularly since LC neurons and their NA containing axons are lost in AD and may also innervate the retina. A common loss of noradrenergic innervation of RGCs and central neurons in AD might explain glaucomatous loss of RGCs in AD. Furthermore, systemic treatment of AD patients with α2-adrenergic receptor agonists may slow both RGC and central neuronal apoptosis in AD by acting on α2-adrenergic receptors that are retained by surviving neurons.

### Example 1

### Methods:

### Open-Field Test

Each animal is placed in a clear plastic open field box with two rows of photo-beams mounted on the sides to distinguish be5tween horizontal (i.e. distance traveled) and vertical (i.e. "rears") movements. Ambient conditions are low noise and dim lighting. The animal's movements within the box are measured for 5 minutes, and calculated from records of the number and type of "beam-breaks" or photo-beam crossings. On the last Open Field test, only rears are counted, and classified as either "supported" or "unsupported". Supported rears are when an animal places at least one forelimb on the side wall of the box when a rear is recorded. In an unsupported rear the mouse is supported solely by hindlimbs. These rears are distinguished by videorecording. The number of unsupported rears is the most reliable measure of dopamine neuron loss in this test.

### Tail Hanging test

Mice are hung by their tails 3 times each, for approximately 10 seconds each time. Each mouse is hung by the base of its tail about 30 cm above the surface of a table until the mouse turns either left or right. A left turn is given a score of 0 and a right turn is given a score of 1.

Forelimb placement is also noted during tail hanging. The placement of the limbs is given a score on a 4 point scale. Extended limbs, or those placed above the head are given a score of 0. Limbs which are clasped or held against the body are given a score of 3. Scores of 1 or 2 are assigned for relative stages between the two extremes. Hindlimb placement is also scored as below.

### Nest Building

4 mice of the same gender from one group are placed in a plastic tub. Eight strips of paper towel are placed in a neat pile at the front of the tub. The nests build from the paper towel are scored at 24 hours, 48 hours, 72 hours and 96 hours following treatment. Scores are assigned as follows: 0 = paper shredded and formed into a full nest with overhead cover; 1 = paper shredded and formed into a full nest without overhead cover; 2 = paper slightly shredded or chewed and loosely gathered into one area; 3 = paper slightly chewed with no apparent gathering; and 4 = no apparent gathering.

### Neuron Counting

Between 55 and 60 day post MPTP injection, the mice are sacrificed using sodium nembutol. Mouse brains are perfused with phosphate buffered saline followed by Lana's fixative (paraformaldehyde and picric acid). The brains are removed and placed in Lana's fixative for 7 to 10 days. The brains are then sectioned coronally at 50 micrometers using a vibratome. And the sections stained with an antibody to tyrosine hydoxylase, the rate limiting enzyme in dopamine synthesis. The section was then examined under microscope at 100X magnification. Tissue slices (-2.9 mm and -3.6 mm posterior to Bregma were selected for cell counting in the SN and VTA. These sections are at the midpoint of the rostral half and the midpoint of the cauldual half of SN, respectively. Each TH-labeled cell that is clearly visible that has between 2 and 6 neurites is considered a neuron. An overall average count for each animal is calculated from the four sections (rostral and caudal, left and right). An average is obtained for the four sections. Separate analyses are performed on the neuron counts from SN and VTA. These counts are analyzed using a repeated measures ANOVA, following by tests of the between groups effect using Fisher's HSD method.

### Experimental Procedure

Mice that receive systemic injections of the pyridine toxin 1-methyl-4-phenyl-1,2,3,6-tetrahydropyridine (MPTP) selectively lose large numbers of dopaminergic neurons in the substantia nigra (SN) and the ventral tegmental area (VTA). Loss of dopamine cells in the SN mimics the clinical condition seen in Parkinson's disease. Loss of such cells in the VTA may contribute to the cognitive deficits seen in Parkinson's and Alzheimer's disease, due to these neurons' projections into the frontal cortex.

30 C57B1/B6 type mice (8-12 weeks old) are allowed to acclimate for 12-14 days before experimental use. The mice are then randomly assigned to the following groups: MPTP plus DMSO vehicle; vehicle alone, and MPTP plus brimonidine(3mg/kg/day).

In the presently described assays, each mouse is then given an initial Open-Field test and a Tail Hanging test. The Open Field test is the most frequently used behavioral assay of MPTP-treated mice, and appears to be sensitive to loss of dopaminergic input from the substantia nigra. The Tail-Hanging test is sensitive to direct striatal damage; the Nest Building test is sensitive to loss of striatal input from the frontal cortex.

Two out of the 3 groups of mice receive an infusion of a test compound (or of vehicle containing no compound). These infusions are administered via subcutaneous implanted osmotic mini-pump over a 14 day period at a flow of 0.25 microliters/hour. Three days following implantation of the pumps, the mice are given the Open Field and Tail Hanging tests, along with the group of control mice, who are not implanted with mini-pumps. Immediately following the tests, the pump containing mice were given a 40 mg/kg injection of MPTP subcutaneously. All groups are then given the Open Field and Tail Hanging tests at 10-12 days and 30-40 days following MPTP treatment. The Open Field, and Tail Hanging tests are given at 50-55 days post MPTP treatment.

For all behavioral tests, the results are first analyzed using a repeated measures ANOVA, following by tests of the between groups effect using Fisher's HSD method.

### Results

### Open Field test

Prior to MPTP treatment there is no significant difference among the 3 groups in distance traveled or number of rears.

The vehicle group is significantly more active than controls at 10 and 30 days post MPTP treatment. The brimonidine treated mice show no alteration in this increase of activity; thus there is no significant difference between this group and the vehicle group.

MPTP treatment appears to cause a reduction in the total number of rears at 10 days post MPTP. At 30 days there is no MPTP effect on total rearing (as compared with the vehicle group), and only a slight reduction in rearing versus the control group.

The vehicle group make fewer unsupported rears than normal mice. There is no effect of either MPTP or the compounds on supported rearing.

### Tail Hang test

No significant group differences are observed in the tail hang test prior to MPTP treatment, and none are seen post MPTP in the hindlimbs. MPTPO significantly impaired forelimb extensions at all three post-lesion time points. Thus, this impairment is not reversed over time. Brimonidine does not reduce this impairment during the period that the compound is administered. However, brimonidine does tend to reduce the impairment when measured post-dosing (i.e. after 14 days of compound administration).

### Neuron Count

In the substantia nigra, MPTP-treated animals have 58% fewer neurons than untreated animals. However those that receive brimonidine have significantly less neuron loss.

| **Treatment Group** | **Average Neuron Count (statistical error)** |
|---|---|
| *Control* | 60 (± 5) |
| *Vehicle* + *MPTP* | 25 (± 2) |
| *Brimonidine* | 32 (± 1) |

In the Ventral Tegmental area, MPTP was found to result in an average decrease of 28% fewer neurons than in the Control group. Brimonidine reduced this loss by an average of about 10%.

The examples above illustrate a preferred embodiment of the invention, and are not intended to restrict the scope thereof. The invention is defined by the claims that conclude this specification.

## Claims

1. Use of brimonidine or a pharmaceutically effective salt thereof for the manufacture of a medicament for treating a neurodegenerative condition of the brain of a mammal.

2. Use according to claim 1 wherein said composition is to be administered to the brain of said mammal by systemic delivery.

3. The use according to claim 2 wherein administration of said composition is effective to prevent death or degeneration of neurons projecting to or from an area of the brain of said mammal selected from the group consisting of the ventral tegmental area , the locus ceruleus and the substantia nigra.

4. The use according to claim 1 wherein said neurodegenerative condition is Parkinson's disease.

5. The use according to claim 1 wherein said neurodegenerative condition is Alzheimer's disease.

## Patentansprüche

1. Verwendung von Brimonidin oder einem pharmazeutisch effektivem Salz hiervon zur Herstellung eines Medikaments für die Behandlung eines neurodegenerativen Zustands des Gehirns eines Säugers.

2. Verwendung gemäß Anspruch 1, worin die Zusammensetzung an das Gehirn des Säugers durch systemische Zuführung verabreicht werden soll.

3. Verwendung gemäß Anspruch 2, worin die Verabreichung der Zusammensetzung effektiv ist, um dem Absterben oder der Degeneration von Neuronen vorzubeugen, die in einen Bereich des Gehirns des Säugers, der ausgewählt ist aus der Gruppe bestehend aus dem ventralen Tegmentalbereich, dem Locus ceruleus und der Substantia nigra, hineinführen oder daraus herausführen.

4. Verwendung gemäß Anspruch 1, worin der neurodegenerative Zustand Parkinson-Krankheit ist.

5. Verwendung gemäß Anspruch 1, worin der neurodegenerative Zustand Alzheimer-Krankheit ist.

## Revendications

1. Utilisation de brimonidine ou de son sel pharmaceutiquement efficace pour la fabrication d'un médicament destiné au traitement d'une maladie neuro-dégénérative du cerveau d'un mammifère.

2. Utilisation selon la revendication 1, dans lequel ladite composition doit être administrée au cerveau dudit mammifère par délivrance systémique.

3. Utilisation selon la revendication 2, dans laquelle l'administration de ladite composition est efficace pour empêcher la mort ou la dégénérescence des neurones faisant saillie vers et à partir d'une zone du cerveau dudit mammifère choisi dans le groupe consistant en la zone de la calotte ventrale, le locus caeruleus et la substance noire.

4. Utilisation selon la revendication 1, dans laquelle ladite maladie neuro-dégénérative est la maladie de Parkinson.

5. Utilisation selon la revendication 1, dans laquelle ladite maladie neuro-dégénérative est la maladie d'Alzheimer.
